# EUROPEAN PATENT SPECIFICATION

(11) **EP 2 710 903 B1**
(45) Date of publication and mention of the grant of the patent: **25.10.2017**
(21) Application number: 12861398.1
(22) Date of filing: 05.07.2012
(51) Int. Cl.: A61K 35/64, A23L 21/20

(54) **METHOD FOR EXTRACTING DRONE BEE LARVAE WITH MAXIMUM BIOLOGICAL VALUE**
VERFAHREN ZUR EXTRAKTION VON BIENENDROHNENLARVEN MIT MAXIMALEM BIOLOGISCHEM WERT
PROCÉDÉ DE SÉLECTION DE COUVAINS MÂLES AYANT UNE VALEUR BIOLOGIQUE MAXIMALE

(30) Priority: 29.12.2011 RU 2011153905
(43) Date of publication of application: 26.03.2014
(73) Proprietor: Obshestvo S Ogranichennoj Otvetstvennostju "PARAFARM", Penza 440026 (RU)
(72) Inventor: TRIFONOV, Vyacheslav Nikolaevich, Zarechnij Penzenskaya obl. 442960 (RU); ELISTRATOVA, Julia Anatoljevna, Penza 440026 (RU); ELISTRATOV, Konstantin Gennadievich, Penza 440061 (RU); KURUS, Natalia Vyacheslavovna, Penza 440026 (RU); HOMYKOVA, Irina Vladimirovna, Penza 440003 (RU); ELISTRATOVA, Tatyana Viktorovna, Penza 440018 (RU); BURMISTROVA, Lilia Alexandrovna, Rybnovskiy raion Ryazanckaya obl. 391134 (RU); BUDNIKOVA, Natalya Valentinovna, Rybnovskiy raion Ryzanckaya obl. 391134 (RU)
(74) Representative: Engel, Christoph Klaus
(86) International application number: PCT/RU2012/000541
(87) International publication number: WO 2013/100802

(56) References cited:
- RU-C1- 2 395 289
- RU-C1- 2 414 229
- RU-C2- 2 287 334
- BUDNIKOVA N.V.: 'Biologicheski aktivnye soedineniia v trutnevom rasplode' ZH.PCHELOVODSTVO 2009, pages 1 - 2, XP008172453 Retrieved from the Internet: <URL:http://www.beekeeping.orc.ru/Arhiv/a20 09/n609 52.htm>

## Description

The invention relates to the pharmaceutical and food industry, to medicine, specifically to biologically active dietary supplements (BADS) to food, and describes the process of selection of drone larvae for obtainment of the drone brood.

In Romania, it was patented the method of obtainment of biologically active product from larvae of drones or working bees or queens (Patent RO Nº 74872/1980) that is biologically active substance for obtainment of medicines and cosmetic means. It is known the patent RU Nº 2402920 "Method of preparation of the fodder supplement from drone larvae for increase of dogs organisms resistance by parasitosis". It is known the patent RU Nº 2258522 "The method of preparation making from drone larvae for stimulation of animal organism". It is known the patent RU Nº 2395289 "The method of making of biogenic stimulator from the larvae of drone brood of bees". It is known the patent Nº 2245155 COMPOSITION OF THE CANNED HOMOGENATE OF THE BEES BROOD (DRONE BROOD AND QUEEN LARVAE). In the Romanian patent RO Nº74872/1980 it is said that the larvae are collected before sealing of cells with larvae at 10^{th} day after eggs laying. In the patent RU Nº 2258522 it is said: the preparation is made from the drone larvae of each age. In the patent RU Nº 2402920: It is made collection of larvae 9 - 10 days old. In the patent RU Nº 2395289: the age of drone larvae - 18 - 22 days.

Budnikova, N.V., "Biologically active compound in drone brood" shows a method for extracting drone larvae in order to produce drone brood. The document mentions the biological value of drone brood in various stages of growth.

During numerous works by definition of biological activity of drone brood we have established the term of the drone larvae selection for processing.

Bee queen lays unfertilized eggs, from which drones develop. The period of embryonic development (the stage of egg) forms 3 - 3,5 days. By larvae withdrawal from the eggs, post-embryonic development begins that is accompanied by complete metamorphosis with passing of the stages of larva, prepupa, pupa and imago. Metamorphosis is regulated by hormones. The stage of larva (near 7 days) is characterized by intensive nutrition. From the 7^{th} to 10,5^{th} days (the counting is made from the zero day) the larvae of drones finish nutrition, straighten, settling by the head end to the aperture of cell, which is sealed by bees with porous wax lid, the stage of prepupa begins (from 10^{th} to 14^{th} day). In prepupae fat body is well developed, organs of vision, sense of smell, pigmentation are absent. During all the larval stage formation of testicles proceeds, at the end of which testicles reach size of the adult insect. From 15^{th} to 24^{th} days (from the moment of the egg laying) - stage of pupa, which externally resembles adult drone. After termination of the stage of pupa the adult drone quits the cell and comes out on the surface of a honeycomb.
Temporal interval in each age period is explained by the force of the bee family and its provision by the food. For this reason it is impossible to provide the instruction for the bee keeper that it is necessary to collect larvae just in the given day, since biological value of a drone brood retrieved will be different in different families of bees. We have established external sign of drone brood selection: prepupae in the sealed cells, occupying vertical position with absence of pigmentation (not according to the invention) or pupae with colorless or slightly yellowing eyelets. To use the pupae with the expressed pigmentation (eyelets with violet tint, skin covers with yellowish one) in the processing is undesirably. Prepupae, pupae with yellowing eyelets have highest contains of prohormones. The given sign is very technological, because it permits to select visually the necessary pupae and permits to reject costly investigations of the collected drone brood on biological activity. The given sign permits to standardize the collected drone brood by the acting substances.

### Example 1)

If immature larvae are collected, i. e. before the sign mentioned, the larvae will not be settled vertically, but will be twisted as a worm. Immature larvae give lesser amount of the decenoic acids.

### Example 2)

The larvae with blue eyelets contain excrements and also low contain of hormones: estradiol, prolactin, progesterone. Composition and properties of the drone brood, harvested by the given method, also have differences. Thus, contain of the main sexual hormones (testosterone, progesterone, prolactin, esradiol) in 100 g of the drone brood, harvested by the method claimed, defined by radio-immune methods with the use of standard sets "Immunotech" (Czech Respublic) on radioisotopic apparatuses was maximal (see table 1). By harvesting of drone brood according to the method claimed there are reached also maximal indexes by contain of unsaturated compounds (by the lowermost index of oxidability), decenoic acids (table 2). By the harvesting of drone brood by other method the given indexes have been lowered.

**Contain of testosterone, progesterone, prolactin and estradiol in drone brood by the method claimed and by another method**

| Indexes | Drone brood by the example 1 | Drone brood by the method claimed |
|---|---|---|
| Testosterone, nmol/100 g | Less than 0.292 | From 0.292 to 0.322 |
| Progesterone, nmol/100 g | Less than 42.36 | From 42.63 to 60.01 |
| Prolactin, nmol/100 g | Less than 344.6 | From 344.6 to 475.4 |
| Estradiol, nmol/100 g | Less than 431.2 | From 431.2 to 847.9 |

**Composition and properties of the drone brood, obtained by the method claimed**

| Indexes | Drone brood by the example 1 | Drone brood by the method claimed |
|---|---|---|
| Contain of unsaturated compounds (by index of oxidation, c) | More than 10.0 | Less than 10.0 |
| Mass part of decenoic acids, % | Less than 3.0 | More than 3.0 |
| Mass part of raw protein | Less than 26% | From 28.0 to 32.0 |
| Concentration of hydrogen ions (pH) | Less than 6.1 | From 6.1 to 6.8 |

## Claims

1. Method of selection of drone larvae for preparation of drone brood, including: harvesting of pupae with colorless or slightly yellowing eyes from the cell sealed, permitting to reach the contain of testosterone, progesterone, prolactin and estradiol in the drone brood with characteristics:
1) Testosterone not less than 0.292 nmol/100 g;
2) Progesterone not less than 42.63 nmol/100 g;
3) Prolactin not less than 344.6 nmol/100 g
4) Estradiol not less than 431.2 nmol/100 g;
5) Unsaturated compounds, indexes of oxidability less than 10.0 c;
6) Decenoic acids not less than 3.0 %
7) Raw protein from 28.0 to 32.0 %
8) Concentration of hydrogen ions (pH) from 6.1 to 6.8.

## Patentansprüche

1. Verfahren zur Selektion von Drohnenlarven zur Gewinnung von Drohnen-Brut, umfassend: Entnahme von Puppen mit farblosen oder leicht vergilbenden Augen aus der versiegelten Zelle, wodurch die Erlangung eines Gehalts an Testosteron, Progesteron, Prolaktin und Estradiol in der Drohnenbrut ermöglicht wird mit den Eigenschaften:
1) Testosteron nicht weniger als 0.292 nmol/100 g;
2) Progesteron nicht weniger als 42.63 nmol/100 g;
3) Prolaktin nicht weniger als 344.6 nmol/100 g
4) Estradiol nicht weniger als 431.2 nmol/100 g;
5) Ungesättigte Verbindungen, Oxidationsindizes weniger als 10.0 c;
6) Decenoesäuren nicht weniger als 3.0 %
7) Rohprotein von 28.0 bis 32.0 %
8) Konzentration von Wasserstoffionen (pH) von 6.1 bis 6.8.

## Revendications

1. Procédé de sélection de larves de faux bourdon pour la préparation de couvain de faux bourdon, consistant à : récolter des nymphes ayant des yeux incolores ou légèrement jaunissant à partir de la cellule scellée, permettre d'atteindre la quantité de testostérone, de progestérone, de prolactine et d'oestradiol dans le couvain de faux bourdon avec les caractéristiques suivantes:
1) Testostérone pas moins de 0,292 nmol/100 g ;
2) Progestérone pas moins de 42,63 nmol/100 g ;
3) Prolactine pas moins de 344,6 nmol/100 g ;
4) Estradiol pas moins de 431,2 nmol/100 g ;
5) Composés insaturés, indexes d'oxydabilité inférieurs à 10,0 c ;
6) Acides décénoïques pas moins de 3,0 %
7) Protéine brute comprise entre 28,0 et 32,0 %
8) Concentration en ions hydrogène (pH) comprise entre 6,1 et 6,8.
